# FASCICULE DE BREVET EUROPEEN

(11) **EP 0 462 008 B1**
(45) Date de publication et mention de la délivrance du brevet: **30.11.1994**
(21) Numéro de dépôt: 91401567.2
(22) Date de dépôt: 13.06.1991
(51) Int. Cl.: A61F 2/02

(54) **Dispositif filtrant destiné à la prévention des embolies**
Filter für Blutgerinsel
Blood dot filter

(30) Priorité: 15.06.1990 FR 9007535
(43) Date de publication de la demande: 18.12.1991
(73) Titulaire: ANTHEOR, F-86130 Jaunay Clan (FR)
(72) Inventeur: Metais, Joel, F-86420 Berthegon (FR)
(74) Mandataire: Hubert, Philippe

(56) Documents cités:
- EP-A- 0 121 447
- EP-A- 0 188 927
- EP-A- 0 348 295
- FR-A- 2 573 646

## Description

La présente invention a pour objet un dispositif filtrant à centrage automatique, destiné à la prévention des embolies.

On sait que les embolies résultent de l'oblitération d'un vaisseau sanguin par un caillot ou un corps étranger véhiculé par le sang, jusqu'au lieu où le calibre est insuffisant pour permettre son passage.

Pour prévenir les embolies, on a déjà proposé de nombreux dispositifs filtrants, de configurations variées, destinés à être placés sur le trajet sanguin pour la retenue des caillots ou corps étrangers susceptibles de provoquer l'embolie.

De tels dispositifs sont notamment connus par les documents US-A-3.952.747 ou FR-A-2.573.646.

Ces dispositifs connus comportent généralement des bras élastiques disposés régulièrement autour d'un axe central, et dont certains sont pourvus à une extrêmité d'un crochet destiné à permettre un ancrage dans la paroi du vaisseau.

Par ailleurs, le dispositif révélé par le document FR-A-2.573.646 est conformé pour assurer un centrage automatique du filtre lors de sa mise en place ; l'axe du filtre étant sensiblement confondu avec l'axe de la veine dans laquelle il est disposé.

On sait également que les dispositifs filtrants sont généralement mis en place dans la veine de façon temporaire ou permanente, voir par exemple le filtre illustré dans le document EP-A-0 348 295, par voie percutanée haute (jugulaire) ou basse (fémorale), au moyen de divers accessoires (notamment gaine et poussoir). En outre, ces filtres sont généralement commercialisés dans un emballage stérile avec leurs accessoires de mise en place.

L'introduction par voie haute a été utilisée de façon majoritaire (environ 90 % des cas) pendant de nombreuses années. Il semble, à l'heure actuelle, que l'on assiste à une inversion de tendance.

Il est à noter qu'en raison de leurs formes, non symétriques, les dispositifs filtrants actuellement utilisés nécessitent pour leur mise en place des accessoires différents, selon la voie choisie pour la mise en place dans la veine.

Or, il arrive en pratique que la voie d'introduction initialement choisie par l'opérateur chargé de la mise en place, s'avère inappropriée, ce qui nécessite le recours à deux jeux différents d'accessoires, et parfois même, l'utilisation d'un deuxième filtre.

La présente invention a pour but de résoudre le problème technique consistant en la fourniture d'un dispositif filtrant d'une nouvelle conception, à centrage automatique qui puisse être mis en place de façon temporaire ou permanente, indifféremment par voie haute ou basse à l'aide d'un même jeu d'accessoire, en garantissant une efficacité au moins comparable à celle des filtres existants.

La solution, conforme à la présente invention, pour résoudre ce nouveau problème technique consiste en un dispositif filtrant à centrage automatique destiné à la prévention des embolies, caractérisé en ce qu'il comprend un ensemble de bras élastiques, incurvés, identiques et réunis ensemble à leurs extrêmités, lesdits bras, à l'état déployé de service, étant angulairement répartis de façon sensiblement régulière (disposés deux à deux successivement en sens inverse).

Cette solution permet de résoudre le problème technique précédemment énoncé, d'une manière extrêmement simple, facile à mettre en oeuvre à l'échelle industrielle.

Selon un mode de réalisation actuellement préféré de l'invention, chaque bras précité comporte une portion convexe dont le sommet est destiné à venir en appui contre la paroi interne de la veine, à l'état déployé de service, ladite portion convexe étant plus proche d'une extrêmité du bras que de l'autre, de sorte que ledit dispositif filtrant prend appui sur la veine par l'intermédiaire de deux séries de points espacées longitudinalement d'une distance pré-déterminée, avantageusement comprise entre 20 et 30 mm.

On obtient ainsi un excellent centrage automatique du filtre lors de sa mise en place dans la veine.

Selon une caractéristique particulière de l'invention, chaque bras est réalisé sous la forme d'une lamelle, en une ou deux parties, obtenue de préférence par laminage d'un fil réalisé en un alliage de qualité médicale, avantageusement un alliage à 40 % de cobalt.

Suivant une variante de réalisation utile pour une mise en place permanente dudit dispositif filtrant conforme à l'invention, chaque bras précité comporte un organe d'ancrage disposé avantageusement entre la partie centrale du bras et le sommet de la portion convexe précitée, de préférence à proximité de ce sommet.

On obtient ainsi un excellent ancrage, par l'intermédiaire de deux séries longitudinalement espacées, de points d'ancrage inversés, permettant d'empêcher tout mouvement longitudinal du filtre aussi bien dans le sens de circulation du flux sanguin que dans le sens inverse.

Selon une caractéristique particulièrement avantageuse de l'invention, chaque bras précité est revêtu, sur toute sa surface, de préférence à l'exception des surfaces destinées à venir en contact avec la paroi interne de la veine, d'une fine couche régulière de carbone à structure amorphe.

Il a en effet été découvert, de façon tout à fait surprenante et inattendue, qu'un tel revêtement améliore la thrombogénicité du dispositif filtrant.

L'application de cette découverte n'est pas limitée au seul dispositif filtrant conforme à l'invention ; tout filtre pouvant être revêtu par un tel revêtement.

Selon une autre caractéristique particulière de l'invention, les bras précités sont réunis ensemble à leurs extrêmités par fixation, par exemple par soudure électrique par points, sur une pièce de liaison sensiblement cylindrique creuse.

Avantageusement, les pièces de liaison, disposées aux deux extrêmités des bras, sont de diamètre différent.

L'invention sera mieux comprise, et d'autres buts, caractéristiques et avantages de celle-ci, apparaitront plus clairement à la lecture de la description explicative qui va suivre, faite en référence aux dessins schématiques annexés donnés uniquement à titre d'exemples non limitatifs, et illustrant un mode de réalisation actuellement préféré de l'invention, dans lesquels :
- la figure 1 est une vue en perspective d'un dispositif filtrant conforme à l'invention, mis en place dans une veine ;
- la figure 2 est une vue en bout du dispositif filtrant représenté à la figure 1 ;
- la figure 3 est une vue en élévation du dispositif filtrant représenté à la figure 1 ;
- la figure 4 est une vue agrandie du détail A de la figure 3 montrant une extrêmité du dispositif filtrant ;
- la figure 5 A est une vue agrandie du détail B de la figure 3 illustrant un premier mode de réalisation de l'organe d'ancrage d'un bras du dispositif filtrant ;
- la figure 5 B est une vue identique à la figure 5-A illustrant un deuxième mode de réalisation de cet organe d'ancrage ;
- la figure 5 C est une vue indentique à la figure 5-A d'un troisième mode de réalisation de cet organe d'ancrage ;
- la figure 5 D est une vue de dessus de la figure 5 C ;
- la figure 6 A est une vue de la partie de filtre représentée à la figure 5 A, mis en place à l'intérieur d'une gaine d'introduction ;
- la figure 6 B est une vue de la partie de filtre représentée à la figure 5 A mis en place à l'intérieur d'une veine ; et
- la figure 7 est une vue de détail en coupe longitudinale illustrant la mise en place du dispositif filtrant conforme à l'invention à l'intérieur d'une gaine d'introduction.

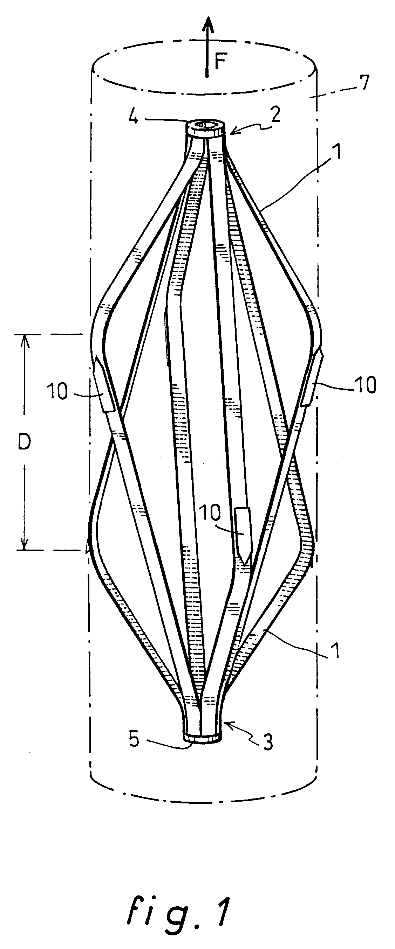

En se référant tout d'abord aux figures 1 à 3, un dispositif filtrant (ci-après "filtre") conforme à la présente invention est essentiellement constitué par un ensemble de bras élastiques 1, par exemple au nombre de 6, incurvés, identiques, disposés deux à deux successivement en sens inverse, et réunis ensemble à leurs extrêmités 2, 3, notamment par fixation, par exemple par soudure électrique par points, sur deux pièces de liaison 4, 5.

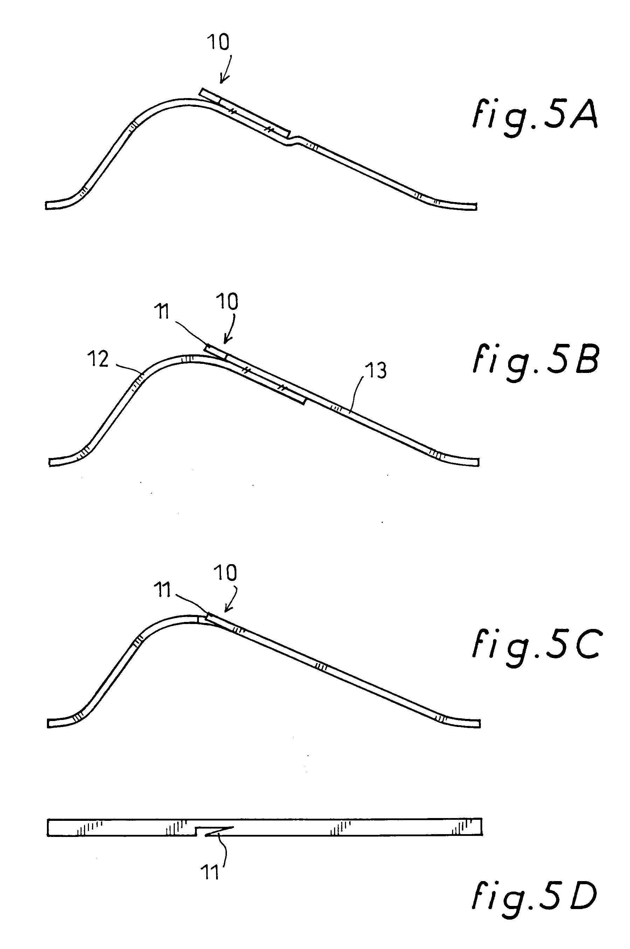

Comme le montre la figure 4, chaque pièce de liaison est constituée par une pièce sensiblement cylindrique creuse ; les pièces de liaison 4 et 5, présentant des diamètres internes différents pour des raisons qui apparaîtront par la suite.

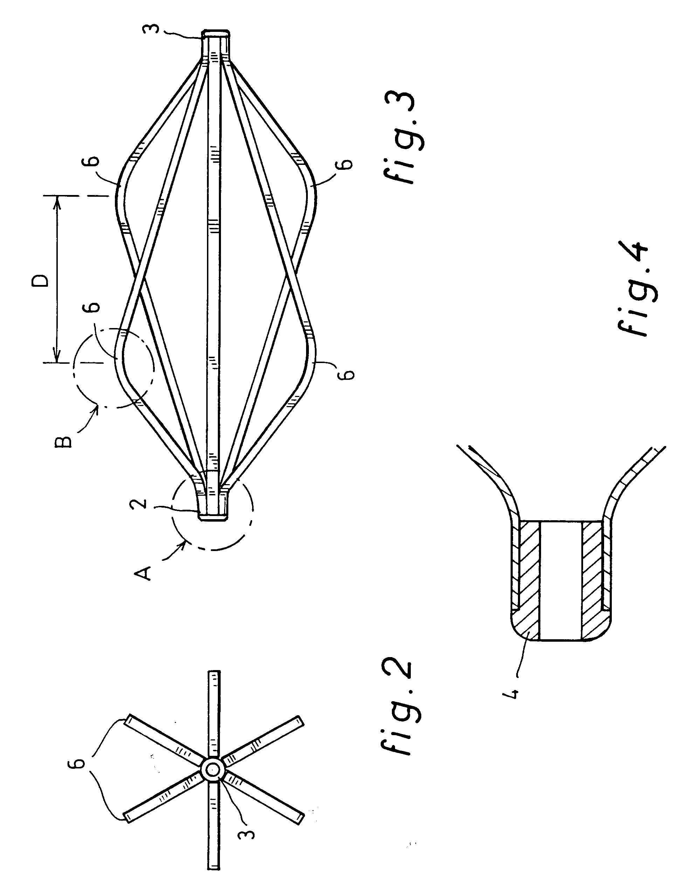

Comme le montre la figure 3, les bras 1, à l'état déployé de service, sont angulairement répartis de façon sensiblement régulière, par exemple espacés deux à deux successivement d'environ 60° dans l'exemple représenté.

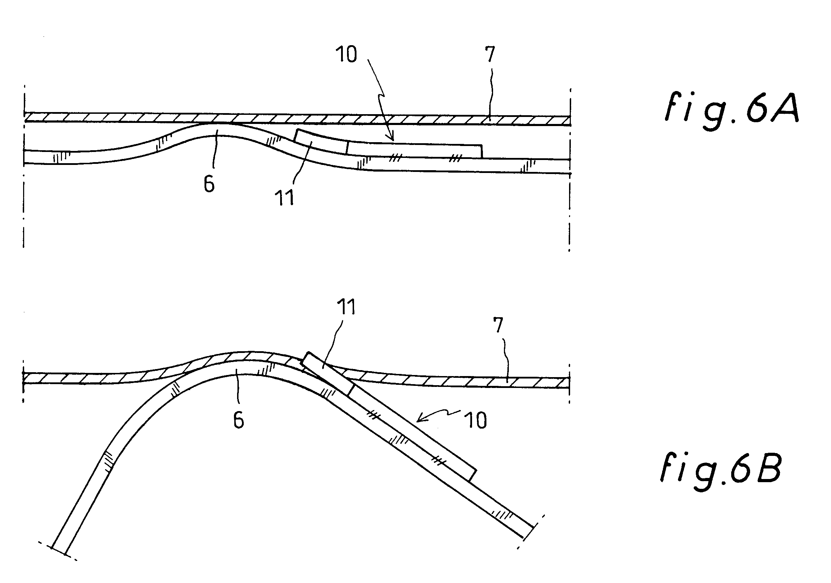

Selon un mode de réalisation actuellement préféré, chaque bras 1 comporte une portion convexe représentée en détail à la figure 6 B, et dont le sommet 6 est destiné à venir en appui contre la paroi interne de la veine 7, à l'état déployé de service.

Cette portion convexe, qui peut être par exemple réalisée par galetage, est plus proche d'une extrêmité du bras 1 que de l'autre, de sorte que les bras étant identiques et montés deux à deux successivement en sens inverse, ledit dispositif filtrant prend appui sur la veine, au niveau des sommets 6, par l'intermédiaire de deux séries de points espacées longitudinalement d'une distance D prédéterminée (voir figure 1).

La distance D est avantageusement comprise entre 20 et 30 mm, de préférence entre 20 et 25 mm.

Chaque bras 1 est avantageusement réalisé sous la forme d'une lamelle par laminage d'un fil cylindrique de section adaptée à la section du bras.

Le laminage permet d'obtenir un ruban facile à travailler, à bords réguliers, nets et sans bavure.

Le fil utilisé pour réaliser le bras peut être constitué par un alliage de qualité médicale, avantageusement un alliage amagnétique à 40 % de cobalt, compatible avec les explorations par résonnance magnétique nucléaire, tel qu'en particulier un alliage commercialisé sous la dénomination PHYNOX ® disponible chez IMPHY.

Selon une caractéristique particulièrement avantageuse, afin d'améliorer la thrombogénicité du filtre, chaque bras 1 est revêtu sur toute sa surface, de préférence à l'exception des surfaces destinées à venir en contact avec la paroi interne de la veine, d'une fine couche régulière, par exemple d'environ 1 µm, de carbone à structure amorphe.

Ce dépôt peut être effectué à basse température (environ 100°C) par dépôt en phase vapeur magnétron plan avec cible graphite.

Le fait de conserver à nu, les surfaces des bras destinés à venir en contact avec la paroi interne de la veine, permet d'améliorer la stabilité du filtre lorsque celui-ci est disposé de façon permanente. En effet, quelques jours après la pause, l'endothélialisation se produit et le filtre est définitivement fixé à la paroi de la veine.

Il est à noter qu'un tel revêtement peut être réalisé sur tout filtre, quelque soit sa forme (voire même à tout appareil médical destiné à être en contact de façon permanente avec le sang) en produisant l'effet avantageux mentionné précédemment.

Le dispositif filtrant, conforme à l'invention, peut être conformé pour une introduction permanente ou temporaire dans une veine.

Dans le but d'une filtration permanente, chaque bras 1 comporte un organe d'ancrage, généralement repéré par le repère 10, et représenté en détail, aux figures 5 A à 5 D et 6 A, 6 B.

Chaque organe d'ancrage est disposé avantageusement entre la partie médiane du bras 1 et le sommet 6 de la portion convexe précitée, de préférence à proximité de ce sommet.

Lorsque le filtre est à l'état déployé de service (figure 6 B), chaque organe d'ancrage vient pénétrer localement dans la paroi interne de la veine 7, en assurant ainsi la fixation du filtre.

Les bras étant identiques et disposés deux à deux successivement en sens inverse, on obtient ainsi un excellent ancrage, par l'intermédiaire de deux séries longitudinalement espacées, de points d'ancrage inversés, empêchant ainsi tout mouvement longitudinal du filtre (c'est-à-dire suivant l'axe de la veine) aussi bien dans le sens de circulation du sang (indiqué par la flèche F sur la figure 1) que dans le sens opposé.

Les organes d'ancrage sont également conformés de façon à permettre un glissement sans accrochage du filtre dans la gaine d'introduction servant à la mise en place dudit filtre.

A cet effet, comme le montre la figure 6 A, lorsque le filtre se trouve à l'état allongé dans la gaine d'introduction, les éléments d'ancrage viennent se positionner sensiblement parallèlement à l'axe du filtre, en étant maintenus écartés de la paroi interne de la gaine 11 grâce à la partie convexe du bras.

Selon un mode de réalisation préféré, chaque organe d'ancrage comporte une pointe acérée 11.

Cette pointe 11 peut être rapportée, par exemple par soudure électrique par points, lorsque le bras 1 est réalisé sous forme d'une lamelle monobloc (figure 5 A).

Selon une variante de réalisation, le bras 1 peut être réalisé sous la forme d'une lamelle en deux parties 12, 13, l'une de ces parties (13) comportant une extrêmité taillée en forme de pointe acérée (voir figure 5 B).

Enfin, selon une dernière variante de réalisation, la pointe 11 peut être découpée dans la largeur du bras 1, puis formée dans son prolongement (figures 5 C et 5 D).

Dans le but d'une filtration temporaire, le filtre, conforme à l'invention, est sensiblement identique au filtre utilisé dans le but d'une filtration permanente, à ceci près qu'il ne comporte aucun élément d'ancrage.

En outre, dans le cas où l'on souhaite améliorer la thrombogénicité par un dépôt de carbone, ce dépôt sera réalisé sur toute la surface du filtre.

A titre d'exemple purement illustratif, les caractéristiques dimensionnelles d'un filtre conforme à l'invention sont les suivantes :
- Filtre ouvert : 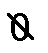 = 31 mm
   long = 60 mm
- Filtre fermé : 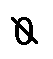 = 2,7 mm
   long = 70 mm
- Largeur des bras : environ 1,20 mm→6 bras
   environ 0,95 mm→8 bras
- Epaisseur des bras : environ 0,10 mm
- Espace entre les points d'appui : 20/25 mm
La mise en place d'un dispositif filtrant, conforme à l'invention, à l'intérieur d'une veine, est connue et de pratique courante. Par exemple, cette mise en place peut être réalisée par poussée dans une gaine d'introduction, préalablement positionnée dans la veine sur laquelle le filtre doit être placé. Cette technique est notamment décrite dans la demande de brevet FR 2.570.288.

Ainsi, les accessoires, généralement nécessaires pour la pose, comprennent :
- une gaine d'introduction comportant une embase à une extrêmité ;
- un poussoir ;
- un guide en J ;
- une aiguille de ponction.

A titre d'exemple, le diamètre interne de la gaine sera d'environ 3 mm (9F) et le diamètre du poussoir de 2,8 mm.

Comme indiqué précédemment, dans le mode de réalisation actuellement préféré du filtre conforme à l'invention, les pièces de liaison, disposées aux deux extrêmités des bras, sont de diamètre différent.

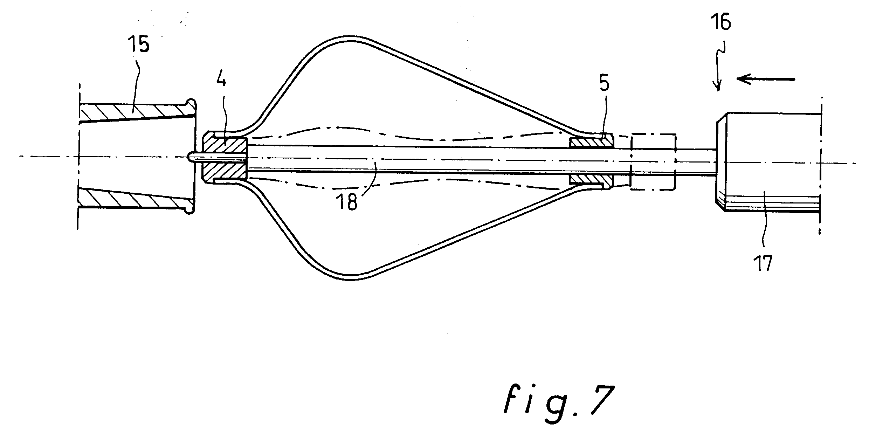

L'intérêt de choisir des diamètres différents apparaîtra à l'aide de la description de la figure 7, illustrant la mise en place du filtre à l'intérieur de la gaine d'introduction.

A la figure 7, on retrouve le filtre à l'état déployé de service et la partie arrière d'une gaine d'introduction 15.

Le filtre est introduit dans la gaine d'introduction 15 à l'aide d'un élément, généralement repéré par le repère 16, composé d'une poignée de manoeuvre 17 et d'une tige à deux diamètres 18.

Le diamètre le plus grand de la tige 18 est compris entre le diamètre de la pièce de liaison 4 et le diamètre de la pièce de liaison 5.

Le plus petit diamètre de la tige 18 est inférieur au diamètre de la pièce de liaison 4, qui est lui même inférieur au diamètre de la pièce de liaison 5.

Ainsi, comme on le comprend, pour sa mise en place dans la gaine, le filtre conforme à l'invention est tout d'abord monté sur la tige 18, qui est conformée pour traverser la pièce de liaison 5 et venir en butée, par sa portion de plus grand diamètre, contre la pièce de liaison 4.

A titre d'exemple, les pièces de liaison 4 et 5 sont respectivement alésées à 1,1 mm et 1,6 mm.

Le filtre ainsi positionné sur la tige 18 est présenté dans l'embase (ou partie arrière de la gaine d'introduction 15).

La poussée sur la poignet 17 de l'élément 16 force le filtre à pénétrer dans l'embase en se rétractant jusqu'à une position sensiblement rectiligne.

Lorsque le filtre est totalement à l'intérieur de la gaine d'introduction 15, la tige 18 est retirée.

On peut alors introduire le filtre dans la veine, à l'endroit du site de filtration, au moyen d'un poussoir.

le larguage du filtre s'effectue par remontée de la gaine sur le poussoir.

Dans le cas d'une filtration temporaire, le positionnement est assuré par un cathéter fixé à demeure à une des deux pièces de liaison. Ce cathéter permet l'introduction et le retrait du filtre par simple traction.

Ainsi, le dispositif filtrant qui vient d'être décrit peut être mis en place, de façon temporaire ou permanente, indifféremment par voie haute ou basse à l'aide d'un même jeu d'accessoires.

Bien entendu, l'invention n'est nullement limitée au mode de réalisation décrit ci-dessus.

Ainsi, le nombre de bras peut varier mais sera de façon générale de six ou huit.

De même, les matériaux utilisés pour la réalisation des bras peuvent être de nature chimique variée.

Le filtre conforme à l'inivention, est notamment destiné à être introduit dans la veine cave, mais peut être également adapté aux dimensions d'autres vaisseaux sanguins.

## Revendications

1. Dispositif filtrant à centrage automatique destiné à la prévention des embolies, caractérisé en ce qu'il comprend un ensemble de bras (1) élastiques, incurvés, identiques, disposés deux à deux successivement en sens inverse et réunis ensemble à leurs extrêmités (2, 3), lesdits bras (1), à l'état déployé de service, étant angulairement répartis de façon sensiblement régulière.

2. Dispositif filtrant selon la revendication 1, caractérisé en ce que chaque bras (1) précité comporte une portion convexe, dont le sommet(6) est destiné à venir en appui contre la paroi interne de la veine, à l'état déployé de service, ladite portion convexe étant plus proche d'une extrémité du bras que de l'autre, de sorte que ledit dispositif filtrant prend appui sur la veine par l'intermédiaire de deux séries de points espacées longitudinalement d'une distance pré-déterminée, avantageusement comprise entre 20 et 30 mm.

3. Dispositif filtrant selon la revendication 1 ou 2, caractérisé en ce que chaque bras (1) précité est réalisé sous la forme d'une lamelle, en une ou deux parties, obtenue de préférence par laminage d'un fil réalisé en un alliage de qualité médicale, avantageusement un alliage à 40 % de cobalt.

4. Dispositif filtrant selon la revendication 2 ou 3, caractériséen ce que chaque bras (1) précité comporte un organe d'ancrage (10) disposé avantageusement entre la partie médiane du bras et le sommet (6) de la portion convexe précitée, de préférence à proximité de ce sommet.

5. Dispositif filtrant selon la revendication 3 ou 4, caractérisé en ce que chaque bras (1) précité est réalisé sous la forme d'une lamelle monobloc, sur laquelle est rapportée, par exemple par soudure électrique par points, une pointe acérée (11) formant moyen d'ancrage précité.

6. Dispositif filtrant selon la revendication 3 ou 4, caractérisé en ce que chaque bras (1) précité est réalisé sous la forme d'une lamelle en deux parties assemblées par exemple par soudure électrique par points, l'une de ces parties comportant une extrêmité en forme de pointe acérée (11) formant moyen d'ancrage précité.

7. Dispositif filtrant selon l'une quelconque des revendications précédentes, caractérisé en ce que chaque bras (1) précité est revêtu, sur toute sa surface, de préférence à l'exception des surfaces destinées à venir en contact avec la paroi interne de la veine, d'une fine couche régulière de carbone à structure amorphe.

8. Dispositif filtrant selon l'une quelconque des revendications 4 à 7, caractérisé en ce que les bras (1) précités sont réunis ensemble à leurs extrêmités par fixation, par exemple par soudure électrique par points, surdeux pièces de liaison (4, 5) sensiblement cylindriques creuses.

9. Dispositif filtrant selon la revendication 8, caractérisé en ce que les pièces de liaison précitées sont de diamètre interne différent.

## Claims

1. Automatically centering filter device intended for preventing embolisms, characterized in that it comprises an assembly of identical, curved in, elastic arms (1), disposed in pairs successively in opposite direction and joined together at their ends (2,3), said arms (1), in the outspread state for use, being angularly distributed in substantially regular manner.

2. Filter device according to claim 1, characterized in that each of said arms (1) comprises a convex portion, of which the apex (6) is adapted to abut against the inner wall of the vein, in the outspread state for use, said convex portion being closer to one end of the arm than to the other, so that said filter device abuts on the vein via two series of points spaced apart longitudinally by a predetermined distance, advantageously comprised between 20 and 30 mm.

3. Filter device according to claim 1 or 2, characterized in that each of said arms (1) is made in the form of a strip, in one or two parts, obtained preferably by rolling a wire produced in a medically compatible alloy, advantageously an alloy containing 40 % cobalt.

4. Filter device according to claim 2 or 3, characterized in that each of said arms (1) comprises an anchoring member (10) advantageously disposed between the median part of the arm and the apex (6) of said convex portion, preferably close to said apex.

5. Filter device according to claim 3 or 4, characterized in that each of said arms (1) is made in the form of a monobloc strip on which a sharp point (11) is added, for example by electric spot welding, said point forming said anchoring member.

6. Filter device according to claim 3 or 4, characterized in that each of said arms (1) is made in the form of a strip in two parts assembled for example by electric spot welding, one of said parts comprising an end shaped as a sharp point (11) forming said anchoring member.

7. Filter device according to any one of the preceding claims, characterized in that each of said arms (1) is coated over the whole of its surface, preferably with the exception of those surfaces intended to come into contact with the inner wall of the vein, with a regular thin layer of carbon of amorphous structure.

8. Filter device according to any one of claims 4 to 7, characterized in that said arms (1) are joined together at their ends by being fixed, for example by electric spot welding, on two substantially cylindrical, hollow connecting pieces (4, 5).

9. Filter device according to claim 8, characterized in that said connecting pieces are of different internal diameter.

## Patentansprüche

1. Filter mit automatischer Zentrierung zur Verhinderung von Embolien, dadurch gekennzeichnet, daß er eine Gruppe identischer elastischer, gebogener Arme (1) aufweist, die paarweise hintereinander in entgegengesetzter Richtung angeordnet sind und an ihren Enden (2, 3) verbunden sind, wobei diese Arme (1) im ausgestreckten Benutzungszustand im wesentlichen gleichmäßig winklig verteilt sind.

2. Filter nach Anspruch 1, dadurch gekennzeichnet, daß jeder Arm (1) einen konvexen Bereich aufweist, dessen Scheitel (6) im ausgestreckten Benutzungszustand dazu bestimmt ist, an die Innenwand der Vene anzuliegen, wobei dieser konvexe Bereich näher an dem einem als an dem anderen Ende des Arms liegt, so daß der Filter mittels zweier, der Länge nach in einem vorbestimmten Abstand angeordneter Serien von Punkten an der Vene anliegt, wobei dieser Abstand günstigerweise zwischen 20 und 30 mm beträgt.

3. Filter nach Anspruch 1 oder 2, dadurch gekennzeichnet, daß jeder Arm (1) in Form einer ein- oder zweiteiligen Lamelle gearbeitet ist, welche vorzugsweise durch Walzen eines Drahts aus einer für medizinische Zwecke geeigneten Legierung erhalten wurde, vorzugsweise aus einer Legierung, die 40 % Kobalt enthält.

4. Filter nach Anspruch 2 oder 3, dadurch gekennzeichnet, daß jeder Arm (1) eine Befestigungsvorrichtung (10) aufweist, die günstigstenfalls zwischen dem Mittelbereich des Arms und dem Scheitel (6) des vorgenannten konvexen Bereichs und vorzugweise nahe dieses Scheitels angeordnet ist.

5. Filter nach Anspruch 3 oder 4, dadurch gekennzeichnet, daß jeder Arm (1) aus einer einteiligen Lamelle besteht, an die eine scharfe Spitze (11), beispielsweise durch Elektropunktschweißung, gesetzt wird, welche die vorgenannte Befestigungsvorrichtung bildet.

6. Filter nach Anspruch 3 oder 4, dadurch gekennzeichnet, daß jeder Arm (1) in Form einer Lamelle aus zwei Teilen gearbeitet ist, welche beispielsweise durch Elektropunktschweißung zusammengefügt werden, wobei einer dieser Teile ein Ende in Form einer scharfen Spitze (11) aufweist, welche die Befestigungsvorrichtung darstellt.

7. Filter nach einem der vorstehenden Ansprüche, dadurch gekennzeichnet, daß jeder Arm (1) auf seiner ganzen Oberfläche mit einer dünnen, gleichmäßigen Schicht aus strukturlosem Kohlenstoff überzogen ist, vorzugsweise mit Ausnahme der zum Kontakt mit der Innenwand der Vene bestimmten Fläche.

8. Filter nach einem der Ansprüche 4 bis 7, dadurch gekennzeichnet, daß die Arme (1) an ihren Enden durch Befestigung an zwei im wesentlichen zylindrischen, hohlen Verbindungsstücken (4, 5), beispielsweise mittels Elektropunktschweißung, verbunden werden.

9. Filter nach Anspruch 8, dadurch gekennzeichnet, daß die vorgenannten Verbindungsstücke unterschiedliche Innendurchmesser haben.
